(19) **Europäisches Patentamt / European Patent Office / Office européen des brevets**

(11) **EP 2 471 483 B2**

(12) # NEW EUROPEAN PATENT SPECIFICATION
## After opposition procedure

(45) Date of publication and mention
of the opposition decision:
**22.02.2023 Bulletin 2023/08**

(45) Mention of the grant of the patent:
**16.10.2013 Bulletin 2013/42**

(21) Application number: **12161435.8**

(22) Date of filing: **01.03.2006**

(51) International Patent Classification (IPC):
**A61B 90/00** (2016.01)

(52) Cooperative Patent Classification (CPC):
**A61B 90/36; A61B 17/1757;** A61B 34/10

(54) **Surgical planning**

Chirurgische Planung

Planification chirurgicale

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **01.03.2005 GB 0504172**

(43) Date of publication of application:
**04.07.2012 Bulletin 2012/27**

(62) Document number(s) of the earlier application(s) in
accordance with Art. 76 EPC:
**06709968.9 / 1 858 430**

(73) Proprietors:
• **KINGS COLLEGE LONDON
London WC2R 2LS (GB)**
• **DePuy International Limited
Leeds, West Yorkshire LS11 8DT (GB)**

(72) Inventors:
• **Penney, Graeme
London, SE3 7PY (GB)**
• **Barratt, Dean
London, WC1E 6BT (GB)**

• **Hawkes, David
Crawley, West Sussex RH10 3AA (GB)**
• **Slomczykowski, Mike
Harrogate, North Yorkshire HG2 9QG (GB)**

(74) Representative: **Murgitroyd & Company
Murgitroyd House
165-169 Scotland Street
Glasgow G5 8PL (GB)**

(56) References cited:

| | |
|---|---|
| **EP-A- 1 348 394** | **WO-A-02/36031** |
| **WO-A-99/59106** | **WO-A1-01/22368** |
| **WO-A2-02/03304** | **WO-A2-2004/008943** |
| **WO-A2-2004/008943** | **US-A1- 2003 185 346** |
| **US-A1- 2004 171 924** | **US-A1- 2004 263 535** |
| **US-A1- 2005 004 451** | |

• **T.J. Hutton et al. "Active shape models for
customised prosthesis designs", AIMDM'99.
LNAI 1620; pp. 448-452, 1999**
• **Office Action by the United States Patent and
Trademark Office for application no: 11/847,652
dated 22 October 2020**

EP 2 471 483 B2

**Description**

The present invention relates to a computer implemented method for automatically planning or generating planning data or information for at least a part of a surgical procedure which can be or is to be carried out on a body part of a patient.

Image guided surgical techniques can make use of patient images in the planning stage, for example in procedures to implant an orthopaedic joint prosthesis to determine the appropriate location for the components of the joint prosthesis.

Commonly, a surgeon will use images of the patient in the planning stage, which will be manipulated by the surgeon in identifying how best to perform the procedure. This process can be time consuming. It can also require considerable skills on the part of the surgeon, with the risk of errors when the process is performed incorrectly.

WO 02/03304 describes the use of statistical shape models to predict the appearance of a patient's face following reconstructive surgery on their facial bones. A surgical treatment plan is set up by moving some facial landmarks in a 2D lateral image of the patient's head so as then to create an instance of a statistical shape model. This provides a predicted 3D soft tissue structure for the patient's face so that the 3D appearance can be predicted.

WO 2004/008943 describes a system and method to more accurately plan and prepare a bone implant grant by using historical data on previously collected bone grafts. Bone measurement data for a specific bone is used to predict the amount of bone likely present using a regression based technique. Hence a bone graft cut and amount can be more accurately predicted. A mathematical model summarising the bone measurement data can be used instead.

The present invention provides a technique for planning a surgical procedure, which makes use of a statistical shape model of the patient's anatomy (for example with reference to predetermined anatomical structures), in to which is incorporated a representation of a component of hardware (which might be, for example, an implant or an instrument) which is to be used in the procedure.

Accordingly, in a first aspect, the invention provides a computer implemented method for creating a statistical shape model incorporating surgical planning information for a body part, as claimed in claim 1.

The method of the present invention has the advantage that it can reduce the requirement for medical professionals involved in surgical procedure planning (who might be surgeons or technicians) to perform analyses of patient image data. This can reduce the computation time needed during the planning stage of a procedure. There can be a reduced requirement for collection of image data. The skill that is required of the user can be less than is required using certain existing techniques. Furthermore, the statistical shape model can be calculated based on data from historical procedures, which can help to optimise the reliability of the calculation. The technique of the present invention can also be used in surgeon training.

The statistical shape model can be based on at least one of X-ray, CT, magnetic resonance imaging (MRI) and ultrasound scan data. The use of CT scan data is particularly preferred. The use of such data to generate a model of a patient's anatomy is known, for example from Lavallee S et al 1995, Computer assisted spinal surgery using anatomy based registration, in Taylor R H et al (Eds) Registration for Computer Integrated Surgery: Methodology, State of the Art, Cambridge Mass, pp 425-449; and from Chan C S K et al, Cadaver validation of the use of ultrasound for 3D model instantiation of bony anatomy in image guided orthopaedic surgery, in Proceedings of MICCAI 2004.

The present invention involves collecting such scan data from numerous patients to establish a data library. Data from the library can be subjected to statistical analysis to generate a statistical shape model which represents the variation in shape of the data in the library in a compact form, *i.e.* using a small number of degrees of freedom. In this way, it is possible based on limited data from the actual patient to build up a reliable image of the patient's anatomy.

Accordingly, the morphing step preferably involves identifying closest scan data from a data library to anatomical data from the patient. The patient anatomical data can be generated using scanning techniques. It can be generated using a pointer tool.

Preferably, the method of the invention includes the step of incorporating in the virtual model a representation of a component of hardware which is to be used in the procedure. The component of hardware can be represented by data in at least five degrees of freedom, ideally six degrees of freedom, in order to define adequately the location and orientation of the component. This information can then be used to derive instructions for the subsequent performance of the surgical procedure.

Examples of hardware components which might be represented in the virtual model include surgical instruments and implants (including components of orthopaedic joint prostheses).

The method should incorporate sufficient data into the virtual model relating to the hardware component as is necessary to establish uniquely the location and orientation of the component. For example, less data might be required in relation to a component which is rotationally symmetrical than is required for other components. For example, data in five degrees of freedom can be sufficient in relation to a symmetrical component such as the acetabular component of a hip joint prosthesis, whereas data in six degrees of freedom can be required in relation to the femoral component of a hip joint prosthesis.

It can be preferred for the technique used to create the patient model to involve principal component analysis.

Preferably, the technique used to create the patient model involves identifying points on the patient image with corresponding points on a database of images, and forming the patient model. The patient model preferably includes the mean model and modes of variation about this mean. The generation of the patient model preferably involves principal component analysis. Typically, the model will consist of up to five modes of variation, with individual variation of each mode determined by the total variance allowed in the model. The precise choice will depend on the number of example datasets that were used to form the model and the accuracy that is required.

A patient specific model is then instantiated using patient specific scan data, for example from pre-operative X-rays or pre- or peri- or intra-operative 3D tracked ultrasound, or a combination thereof. The patient specific model can be instantiated using a tracked pointer tool during the course of the surgical procedure, which is used to contact predefined points on the surface of the bone. The pointer tools can be tracked optically (for example by means of an array of radiation emitters or reflectors and a fixed camera) or magnetically (for example by means of a coil which is embodied in the tool which can be tracked when it moves in a magnetic field). The use of such pointer tools in surgery to generate location data is well known.

Model instantiation is achieved by reconstructing the closest allowable shape that is consistent with the X-ray or ultrasound images. This involves optimising a cost function that is either computed by minimising the distance between corresponding points in the instantiated model and the observed surface points or by matching based on the instantiated scan intensities in the region of the bone and ultrasound or by matching based on the projection of the instantiated scan and the preoperative image data. We have developed an optimisation method that improves robustness and accuracy of the instantiated model. This scheme uses an iterative closest point (ICP) method to compute the distance between corresponding points in the instantiated model and the ultrasound points. The ICP method is described by P Besl and N McKay in their paper "A method for registration of 3D shapes", published in IEEE Trans Pattern Anal Machine Intell, vol 14, pp239-256 (1992). A four layer optimization strategy is used, where two modes are considered at layer 1 (modes 1 and 2) and this is increased to five modes by layer 4 (modes 1 to 5). On each iteration, a Golden Section search is used to optimise the shape within one mode alone, with the weight corresponding to all other modes held constant.

To provide a further constraint in relation to planning a hip joint replacement procedure, the centre of the rotation of the femoral head could be added as an extra point defined in the template CT scan, and propagated to each individual femur using the registration results. The patient model is rebuilt, and the iterative closest point method is run with the centre of the femoral head in the model defined. In practice, this point can be obtained intraoperatively by pivoting the leg around the hip joint and computing the centre of rotation in the same way as that commonly used to calibrate a tracked pointer.

The model, once instantiated comprises an estimate of the scan (for example a CT scan) of the individual patient as well as the components of the plan (a set of points or points and vectors) in the format required by the image guided surgery system. The surgeon will have the opportunity to modify or even to reject the plan proposed by the system. Surgery will then proceed with the surgical plan as if it had been defined interactively by the surgeon.

The instantiated 3D model and plan could also be used in conjunction with post-operative X-rays to provide an automated system of surgical audit. Such an automated system based on preoperative CT scans and post operative X-rays has been proposed by [Edwards et al Proc CAOS 2002]. Such a system would automatically record adherence to and deviations from the surgical plan. This information could be recorded in the patient's notes, to allow comparison with short, medium and long term follow-up. It could also be incorporated into the surgeon's record for use in professional development and skills assessment and it could be used by the hospital or healthcare system for surgical audit.

Examples of surgical procedures to which the technique of the present invention is applicable include orthopaedic joint procedures (for example replacement of hip, knee, shoulder, ankle and elbow joints), peri-acetabular osteotomy, tibial osteotomy, distal radius osteotomy, anterior cruciate ligament reconstruction, osteoid osteoma excision, bone tumour resection, spinal procedures (for example in the placement of pedicle screws), and fracture surgery. The body part can be a part of a bone, for example the part of a bone which is in the vicinity of a joint which is to be replaced. The body part might be an entire bone: for example it can be useful to have scan data for an entire femur when planning a knee joint replacement procedure.

The technique of the invention can be integrated with tracking components so that a hardware component (especially an instrument or an implant) can be tracked during a procedure. The use of tracking components in surgical procedures is well known, for example using optical, radio frequency, ultrasound, electromagnetic and other technologies.

Embodiments of the invention will now be described in detail, by way of example only, and with reference to the accompanying drawings in which:

Figure 1 shows a high level flow chart illustrating the general method of the invention;
Figure 2 shows a flow chart illustrating the general method shown in Figure 1 being applied in a surgical context;
Figure 3 shows a schematic diagram of a hip joint illustrating the incorporation of planning information into a Statistical Shape Model;

Figure 4 shows a schematic diagram of a knee joint illustrating the incorporation of planning information into a Statistical Shape Model;

Figure 5 shows a flow chart illustrating a method of creating Statistical Shape Model data according to the invention and corresponding generally to the first step of Figure 1;

Figure 6 shows a schematic block diagram of a computer assisted surgery system according to the invention in which the Statistical Shape Model of the invention can be used;

Figure 7 shows a flow chart illustrating a method which uses the Statistical Shape Model of the invention;

Figure 8 shows a flow chart illustrating a further method which uses the Statistical Shape Model of the invention; and

Figure 9 shows a schematic block diagram of a computer part of the system shown in Figure 6.

Similar items in different Figures share common reference numerals unless indicated otherwise.

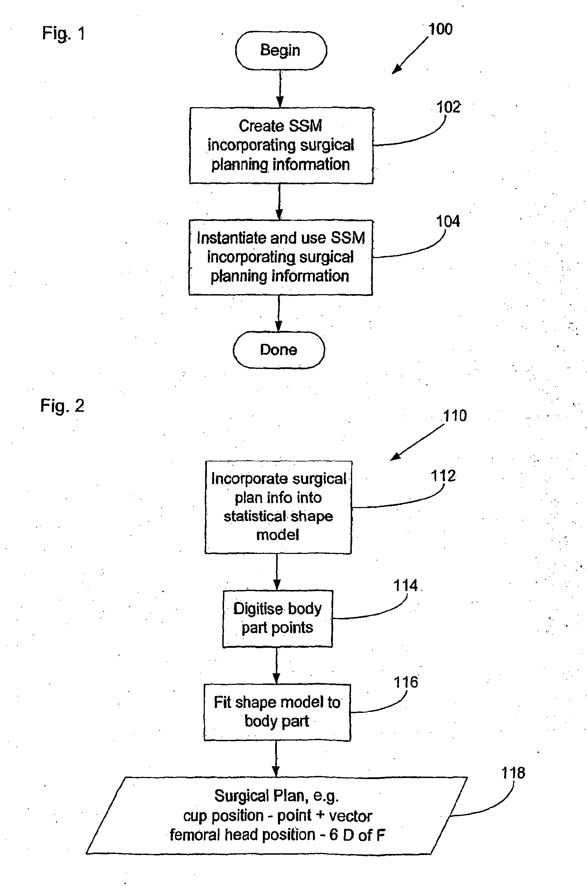

Figure 1 shows a flow chart providing a general overview of the present invention. The method 100 illustrated in Figure 1 includes a first step 102 in which a Statistical Shape Model (SSM) which incorporates information which can be used to at least partially or wholly define a proposed surgical plan. After the SSM has been created, the SSM can be used in a wide variety of applications described below. When a patient specific model of the patient's anatomy is instantiated 104 based on data representing the patient's actual anatomy, the instantiated model also automatically generates surgical planning information which is also specific to the patient's anatomy. That planning information can be used in a wide variety of ways as described below.

In the following, examples of the invention will be described in the context of orthopaedic procedures, but it will be understood that the invention is not limited to application in that area alone. Rather, the invention will be of application in at least all those areas in which, in principle, a SSM can be created.

Before describing the theoretical back ground of the invention, a brief example of the general method 100 will be discussed with reference to Figure 2. The method 110 of the invention includes a first step 112 which corresponds generally to step 102 of Figure 1. In this step information which describes a planned surgical procedure is included in the information from which the statistical shape model will be created. In order to instantiate an actual model and the corresponding plan, patient specific anatomical information is collected 114 using a pointer trackable by a tracking system to "digitise" the surface shape of the body part. An instance of the SSM is then created by fitting the SSM to the collected body part data at step 116. This results in surgical planning data 118 specific to the patients actual anatomy. For example, for a hip replacement operation, this might include the surgical plan for an acetabular cup, defined by the position of the cup relative to the pelvis and the orientation of the cup relative to the pelvis defined by a vector, and the position of the femoral head component specified in six degrees of freedom, *i.e.* three position degrees of freedom and three angular or orientation degrees of freedom.

A more detailed description of the theoretical basis of the invention will be provided before describing various examples of different contexts in which the Statistical Shape Model of the invention can be used. The following description focusses on a point distribution model similar to that proposed by Cootes (1992) in Cootes, T. F., Taylor, C. J., Cooper, D. H., Graham, J., 1992. "Training Models of Shape from Sets of Examples." In: Proceedings of British Machine Vision Conference. pp. 9-18. Also, the planning information is incorporated into the SSM by defining the planning information in terms of a number of points. It will be apparent that other combinations of points can be used in order to define the planning information and that different types of planning information can be incorporated. In the following examples, the planning information relates to the position, orientation and size of surgical implants. However, the planning information does not need to be limited to, or include all or, position, orientation or size. Further, the planning information can relate to different types of components and is not limited to implants. For example, the planning information can relate to the position, orientation, size or type of instruments, tools or other implements used during a surgical procedure. The planning information can also be used for correcting parts of the skeleton to obtain different or optimal biomechanical environments, for example in terms of the stress distribution or peak force location, etc.

In the described embodiment, orientations are represented by two points, with the straight line there through defining the orientation. Orientations can also be derived from combinations of points by carrying out geometric calculations using the points obtained from an instantiation of the model. For example, a plane may be defined by three points and then an orientation be derived from the plane as being the direction normal to the plane. Similarly angles may be derived from the angle subtended by the intersection of two straight lines passing through points obtained from the SSM or from the angle subtended by the intersection of a straight line passing through two points from the model and a plane passing through three points obtained from the model. The derivation of orientation information from the geometry of entities defined by the points obtained from an instantiated model will be understood by a person of ordinary skill in the art in general and within the context of the invention from the discussion herein.

In other embodiments, orientation information can be incorporated in the SSM data by including values for angles directly rather than obtaining angles from the geometry of entities defined by points. However, some care needs to be taken when angles are included as planning data in the SSM data which otherwise comprises data defining the co-ordinates of points. Methods for dealing with this are generally known in the art and are described for example in Morrison, D.F. "Multivariate Statistical Methods" third edition, 1990, McGraw-Hill International Editions, Section 8.2, pages 313-322.

There is a wide variety of shape of the anatomy of bodies, for example the pelvis and femur. This shape variation can be modelled with a SSM which can enable the variation of the shape of bones, or other anatomical structures, *e.g.* organs, to be captured across a population using just a few modes of variation. By incorporating surgical planning data into the SSM the effects of the variation in anatomy across the population on the appropriate plan for that anatomy can also be captured, so that when a model is instantiated for any particular anatomy, the planning information particular to that anatomy will also automatically be generated.

In general a shape can be described using *n* points in d dimension and so can be represented by an nd vector. Planning information can also be described using m points in d dimensions. Therefore, the surface of an anatomical structure, *e.g.* a pelvis or femur, and the three dimensional planning information, can be represented as a 3($n + m$)-element vector x, where

$$x = (x_1,\ldots,x_n,x_{n+1},\ldots,x_{n+m},y_1,\ldots,y_n,y_{n+1},\ldots,y_{n+m},z_1,\ldots,z_n,z_{n+1},\ldots,z_{n+m})^T$$

so that the positions of the *n* points describing the surface of the anatomical structure comprises the points ($x_i$, $y_i$, $z_i$) for I=1 to n, and the *m* points describing the surgical planning information comprises the points ($x_i$, $y_i$, $z_i$) for i=n+1 to n+m. It will be appreciated that the anatomical points and planning points do not need to be sequential in the vector and can be differently arranged in the vector, provided that they have the same arrangement for each vector of the training set. A number of training datasets generated are used to calculate the principal components of shape and planning information variation.

Principle component analysis (PCA) can be used to describe the different modes of variation using a small number of parameters. Data compression is achieved by reducing the number of dimensions without losing the majority of the information. PCA breaks down the data into components, c = ($c_1$, $c_2$, ... $c_{n+m}$) so that they describe the highest amount of variance possible by $n+m$ linearly transformed components.

Firstly the analysis calculates the mean shape $x_{mean}$ and covariance matrix S for the total number of training data sets, s:

$$x_{mean} = \frac{1}{s}\sum_{i=1}^{s} x_i$$

$$S = \frac{1}{(s-1)}\sum_{i=1}^{s} (x_i - x_{mean})(x_i - x_{mean})^T$$

The eigenvectors $\phi_i$ and the corresponding eigenvalues $\lambda_i$ of the covariance S are then computed. The eigenvalues and the corresponding eigenvectors are the solutions of the equation:

$$S\phi_i = \lambda_i\phi_i$$

where $\lambda_i$ is the $i$[th] eigenvalue of S and $\lambda_i \geq \lambda_{i+1}$. Eigenvectors corresponding to the largest eigenvalues describe the most significant modes of variation in the training datasets used to calculate the covariance matrix.

Following PCA, a specific instance of the model can be approximated by

$$x = x_{mean} + \sum_{i=1}^{p} \mu_i\phi_i$$

where x is the approximated instance and $\mu_i$ are the weights for the first p eigenvectors to be used in the approximation.

The calculated eigenvalues show how much variation is covered by each mode, while the corresponding eigenvector is a vector of shape and planning parameters. The higher the eigenvalue, the greater the shape and/or planning change associated with a mode of variation. Linear combinations of the first few modes, for example the first five to ten, can provide an approximation to an individual patients anatomy and the surgical plan configured for that anatomy. Three standard deviations of the mean are typically enough to cover most of the population.

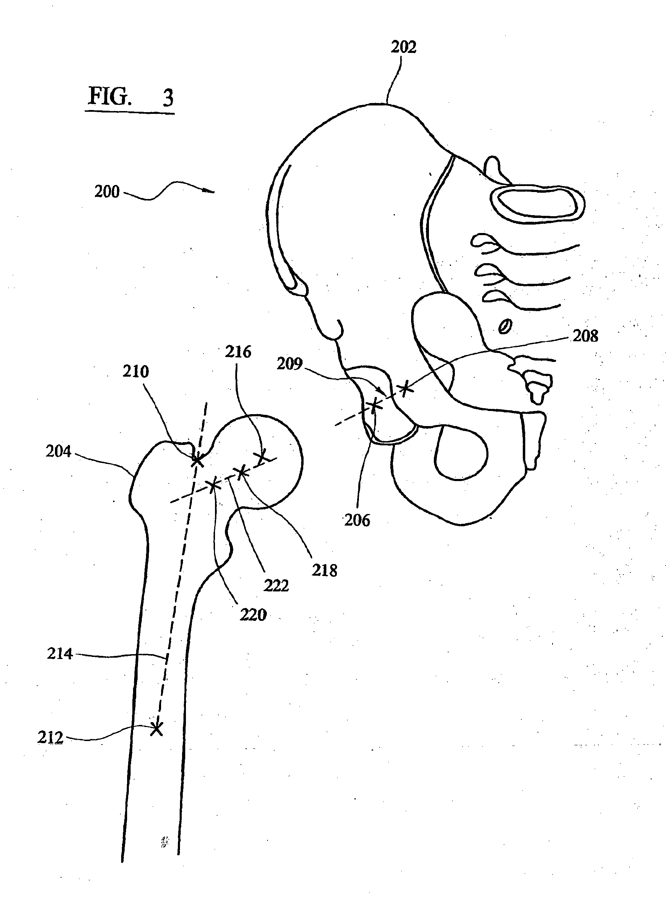

An example of a group of points which can be used to describe a surgical plan will now be given with reference to Figure 3 which shows a diagram of the hip joint 200 and in particular the pelvis 202 and the superior part of the femur 204. A prosthetic hip can include two components, an acetabular cup and a femoral stem. The acetabular cup is generally a hemispherical component and can be provided in a range of different sizes. The acetabular cup is implanted in the acetabulum to provide an articulating surface against which the head of the femoral component can articulate. The femoral stem has a stem part which extends along the intermedullary canal of the femur and has an arm extending therefrom bearing a head which is received in the acetabular cup.

Planning for the acetabular cup component requires the position of the cup relative to the pelvis, the orientation of the cup relative to the pelvis and the size of the cup which should generally match that of the acetabulum of the pelvis. These three planning parameters of the cup can be described using two points associated with the pelvis. The first point 206 is the position relative to the pelvis of the centre of the circular inlet plane or face of the cup. This point describes the planned position of the cup. The second point 208 is the position of a point on the wall of the cup, for which the line 209 through the first and second points is normal to the inlet plane of the cup and which line defines the planned orientation of the cup. The orientation of the cup is generally defined in terms of the angles of inclination and version or anteversion relative to the planes of the pelvis. Hence, the line through the two points 206, 208 describes the planned orientation of the cup. The separation between the two points 206, 208 is the radius of the cup and so describes the planned size of the implant. Therefore, by adding two sets of x, y, z co-ordinates defining the positions of these points to the co-ordinates defining the shape of the pelvis in the vector x, surgical planning information for the acetabular cup implant can be incorporated into the SSM.

If only the acetabular cup is to be replaced, then only this planning information can be incorporated. It will be appreciated that there are other sets of points that can be used to describe the cup planning information. Further, it will be appreciated that other coordinate systems can be used to describe the cup planning information. For example the centre of the cup can be described as an x, y, z position, the orientation of the cup can be described using angles of inclination ($\theta$) and version/anteversion ($\phi$) and the size of the cup by a magnitude of its radius ($|r|$).

More points are used to describe the planning information for the femoral component. A first femoral point 210 identifies the top of the femur. A second femoral point 212 identifies the mid point of the inner diameter of the medullary canal, approximately one third of the way down the length of the femur. The line joining these two points 214 defines the orientation of the longitudinal axis of the superior part of the femur and describing the planned orientation of the stem part of the femoral component.

A third femoral point 216 identifies the centre of the femoral head. The length of the line from the third femoral point 216 which is perpendicular to the direction of the femoral axis, *i.e.* line 214, describes the planned off set for the femoral component.

A fourth femoral point 218 identifies a medial point on the middle of the femoral neck and a fifth femoral point 220 identifies a lateral point on the middle of the femoral neck. The line 222 passing through these points describes the orientation of the arm of the femoral component and the angle subtended by lines 222 and 214 describes the angle of between the arm and stem.

Further planning information is the angle of the arm about the stem axis 214. This can be derived from a group of points as follows. The position of the medial posterior condyle and the position of the lateral posterior condyle and the position of the posterior aspect of the trochanter are identified. These three points define a plane. The angle of the arm axis about the longitudinal femoral axis is defined by the angle subtended between the plane and the line of the arm axis 222. This angle describes the planned orientation of the femoral component relative to the femur.

Further planning information also includes the magnitude of the transverse dimension or 'radius' at at least one position on the neck and the magnitude of transverse dimension or 'radius' at at least one position along the stem. This information can be included as femoral stem components can be angulated in shape which requires one axis for the femoral stem and a point (centre of the femoral head) or two axes, the femoral stem and the neck and the point

Hence, the points described above allow the position of the femoral component to be described, by the centre of the femoral head, the orientation of the femoral component to be described, principally by the neck angle, and the size and shape of the femoral component to be described, principally by the off set and the angle between the arm/neck axis and the stem axis. Therefore, by adding further sets of x, y, z co-ordinates defining the positions of these points to the co-ordinates defining the shape of the pelvis in the vector x, surgical planning information for the femoral implant can be incorporated into the SSM.

In another type of hip arthroplasty, known as acetabular surface replacement or ASR, the femoral head of the femur is removed and replaced with a generally spherical head prosthesis. In order to incorporate surgical planning information into a SSM for an ASR surgical procedure, points associated with the femur are identified from which the planned position of the centre of the femoral head, the planned size or radius of the femoral head and the angle of the neck relative to the longitudinal axis of the femur.

Approaches similar to those described above can be used for a shoulder replacement surgical procedure as the shoulder and hip joints are both essentially a ball and socket type joint. The planning information for a glenoidal component will be similar to that for the acetabular cup and the planning information for the humeral component will be similar to that for the femoral component. It may also be useful to incorporate information allowing additional axes or geometric properties of the scapula to be described, such as the axis of the scapula spinae or the plane of the scapular blade.

The invention can also be applied in other areas such as spinal procedures (in which the planning information would describe the different positions and directions/axes of the spinal components) and fracture/trauma (in which the planning information would describe the recreated bone and the planned size and/or position and/or orientation for an implant, such as a plate or an intermedullary nail).

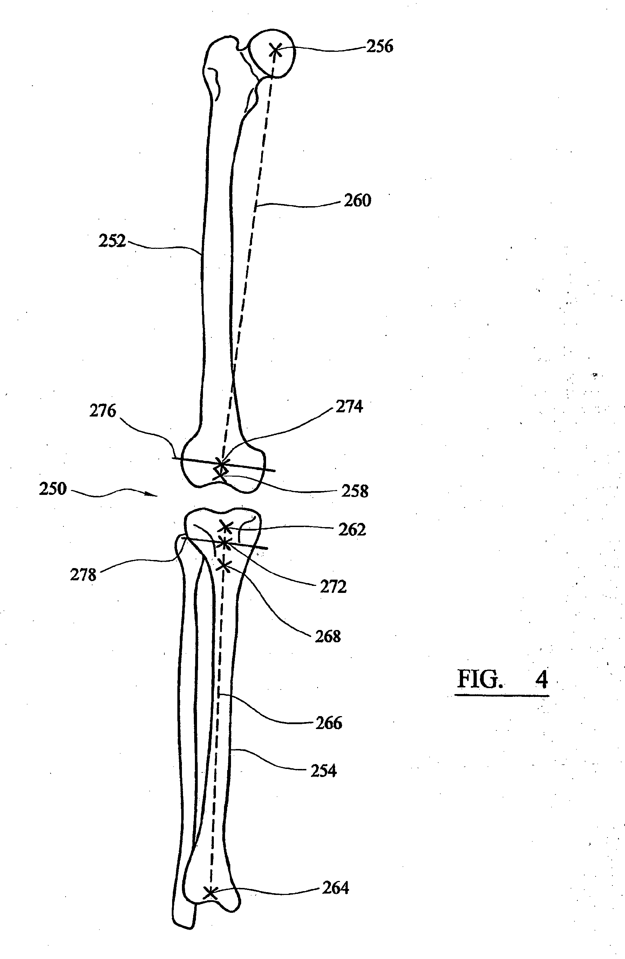

A further example of a group of points which can be used to describe a surgical plan will now be given with reference to Figure 4 which shows a diagram of the knee joint 250 and in particular the femur 252 and the tibia 254. A prosthetic knee can include two components, a femoral component and a tibial component. Often the femoral component has curved articulating surfaces which provide prosthetic condyles and is attached to a resected inferior part of the femur. The femoral component can be provided in a range of different sizes. The tibial component generally includes a tibial tray which is attached to a resected superior part of tibia. A generally concave articulating surface is provided usually by a plastics spacer against which the femoral component articulates. The tibial component can also be provided in a range of different sizes and with spacers of different thicknesses to allow the original joint dimensions to be substantially recreated. Hence, a full surgical plan will generally define the size of the components and the positions and orientations of the components relative to the femur and tibia respectively.

A first femoral point 256 identifies the centre of the femoral head and a second femoral point 258 identifies the femoral notch. The line 260 between these points defines the orientation of the mechanical axis of the femur. A first tibial point 262 identifies the anterior cruciate ligament attachment point and a second tibial point 264 identifies the mid talers. The line 266 between these points defines the orientation of the mechanical axis of the tibia.

A third tibial point 268 identifies the tubercule at which the patella tendon attaches. The position of the medial posterior condyle and the position of the lateral posterior condyle and the position of the posterior aspect of the trochanter of the femur are identified. These three points define a plane relative to which the orientation of the femoral component can be described. The further femoral point 270 identifies the anterior cortex. Femoral anterior cortex point 270 and the medial and lateral posterior condyle points can be used to help determine the planned size of the femoral component.

A further tibial point 272 is identified which defines the planned tibial cut height and a further femoral point 274 is identified which defines the planned femoral cut height. The orientation of the cut for the tibia and femur is defined by the plane 276, 278 perpendicular to the mechanical axis and passing through the cut height point. The positions of medial and lateral extremities of the tibial and femoral cuts are also identified and these points can be used to help determine the planned size of the femoral and tibial implants and in particular their width.

The position of the ACL attachment point establishes the tibial axis (that determines paras valgus, and posterior, anterior slope). The pattela tendon attachment on the tuberositas tibiae establishes the rotation (external, internal) of the tibial component.

Hence, the planned size of the tibial and femoral components can be derived from the points defining the size of the tibia and femur. The planned position of the tibial and femoral components is defined by the tibial and femoral cut heights. The respective planned orientations are defined as being perpendicular to the respective mechanical axes. The respective planned orientations about the mechanical axes can be defined relative to directions of the tibia and femur defined by various combinations of the identified points.

Therefore, by adding further sets of x, y, z co-ordinates defining the positions of these points to the co-ordinates defining the shape of the femur and tibia in the vector x, surgical planning information for a knee replacement surgical procedure can be incorporated into the SSM.

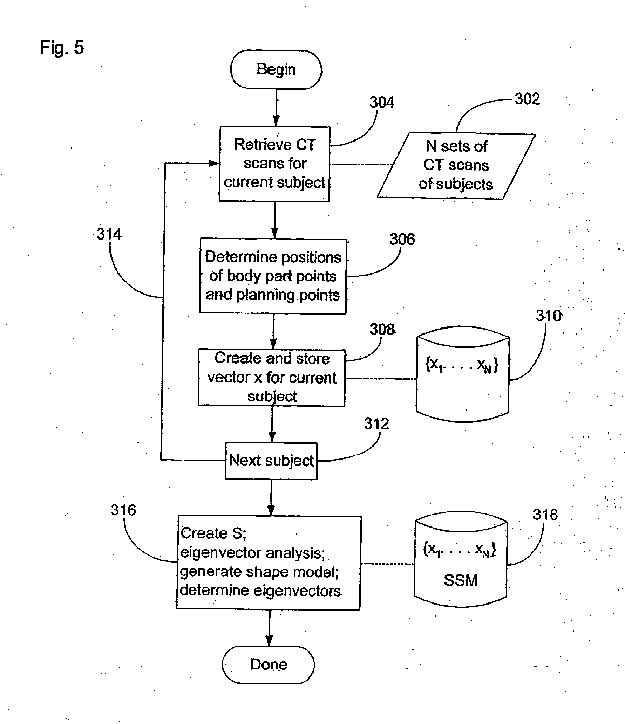

With reference to Figure 5 a computer implemented method 300 for creating the SSM incorporating surgical planning information will be described. The process flow chart shown in Figure 5 illustrating the method 300 corresponds generally to step 102 of Figure 1. Prior to the method beginning sets of CT scan images of a group of N subjects are captured and the CT scan image data 302 is stored in a storage device accessible by the computing device which is used to create the SSM. The training data set 302 does not need to be derived from CT imaging and can be derived from other imaging modalities, *e.g.* Magnetic Resonance Imaging, multiple X-ray views, tracked 2D ultrasound, 3D ultrasound, and only needs to provide data from which a 3D model or representation of the subject's anatomy being modelled can be derived. In one embodiment, the subjects anatomy of the body part of interest does not include any implants or prosthesis. In other embodiments, the subject may be a patient who has previously had a prosthesis implanted so that the imaging also captures images of the implanted prosthesis as well as the patients 'natural' anatomy.

Different groups of subjects can be selected to provide the training set data. Preferably, the group of subjects has a sufficiently wide variation and diversity to cover most of the variations in the general population. However, in other embodiments the subjects for the training data may be selected in order to provide training data for specific circumstances. For example, the subjects may be selected by age, gender, ethnicity, race and any combinations thereof. The subjects may also be selected based on their having a particular condition, disease or other property affecting their anatomy and the surgical plan that would be used. This then provides a SSM which can be used to generate planning data specific to patients also having that condition.

Where the subjects have previously had a surgical procedure, the subjects can be grouped by the type of procedure so that a SSM can be created which is specific to that type of surgical procedure. Similarly, the subjects can be grouped by the type of implant or other components that were used in the procedure so that a SSM can be created which is specific to that type of implant or component. Similarly, the subjects can be grouped by the surgeon or group of surgeons that carried out the procedure so that a SSM can be created which reflects the surgical technique and practices of the surgeon or surgeons. Hence, the expertise and experience of the surgeon or surgeons can be made available to other practitioners either for training or surgical purposes via the SSM. This can also be achieved if no surgical procedure has previously been carried out by identifying the surgical planning points corresponding to the surgeon or surgeons techniques and practices as will be described below.

The method of creating the SSM incorporating surgical planning data begins and at 304 the 3D image data for a first subject is retrieved. Images are created from the image data and can be displayed to a user at step 306. From the images of the subjects a plurality of shape points which describe the characteristic shape of the piece of anatomy are identified and a plurality of points which describe at least some property of the surgical plan are identified. Examples of the points that can be used for hip, knee and shoulder arthroplasty procedures have been described above. The identification of points can be manually carried out by a user identifying points in the images using a cursor and a pointing input device, such as a mouse. The identification of points can also be semiautomatically carried out by the computer using image processing techniques to identify anatomical points and/or surgical planning points in the images and the user manually identifying points. The identification of points can also be entirely automatically carried out by the computer using image processing techniques to identify anatomical points and surgical planning points in the images and the user can then check and verify the identified points and manually make any corrections or changes deemed appropriate.

As mentioned above different types of surgical procedures, different types of surgical components and different types of surgical practices can be captured and incorporated into the SSM. When the subject has not already had the surgical procedure carried out, then the different types of surgical planning information can be captured from the same images so that different SSMs can be created by collecting different sets of surgical planning points. Different protocols or sets of rules can be applied to the point identification process to ensure that the points are collected consistently. For example, an expert surgeon may have a preferred height of tibial cut that should be used during knee surgery for subjects with an extreme or unusual anatomy. Rules capturing that expertise and experience can be provided so that the best surgical practice is incorporated into the SSM. If the subject has already had an implant, then certain groups of points describing the planning information required to reproduce the implants can be identified.

A number of general techniques for capturing the anatomical points can be used. One technique applies a mesh of points over the images for a first subject and then the data sets for the images for the other subjects are registered in order to create the anatomical points. Another technique described by Rueckert et al 2002 (Rueckert, D., Frangi, A. F., Schnabel, J. A., 2003. "Automatic Construction of 3-D Statistical Deformation Models of the Brain Using Nonrigid Registration. pp. 1014-1025) involves carrying out a non-rigid transformation for the images of the different subjects to determine a deformation field.

Once the anatomical points and planning points have all been identified and their co-ordinates determined in the reference frame of the images, then at step 308 then data 310 representing the vector x for the current subject is created and stored in a storage device. Then at 312, if training data remains to be analysed, then processing returns to step 304, as illustrated by process flow line 314. Processing continues to loop until all the training data sets have been analysed so that the data 310 represents the complete set of N vectors $x_1$ to $x_N$ that have been created and stored.

It will be appreciated that Figure 5 is merely schematic and it is not necessary to analyse the training data images sequentially and it is merely intended to show that all the training data images are analysed and in some embodiments that may be done at the same time or in parallel depending on the method used for identifying points.

Once the set of vectors has been created, at step 316 the covariance matrix S is determined using the stored set of vectors. The eigenvectors of the covariance matrix are then determined, as described above so that the shape model can be created. Finally, the number of eigenvectors to be used when instantiating the SSM is determined. typically around 5 to 10 eigenvectors may be sufficient to reproduce the majority of the variation in the training set, but the actual number used can vary depending on the training set and the accuracy required for the intended application. The data representing the SSM 318 which is used instantiate a particular SSM is stored and can then be made available for use by other software applications such as a computer assisted surgery (CAS) application. The method of creating the SSM incorporating surgical planning information is then complete and ends.

A number of applications in which the SSM incorporating surgical planning information can be of particular utility will now be described. These different applications correspond generally to step 104 of Figure 1.

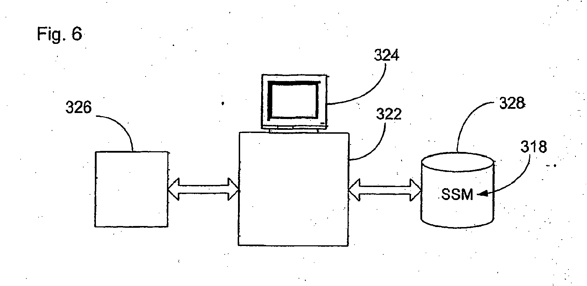

With reference to Figure 6 there is shown a computer assisted surgery (CAS)system 320 with which the SSM of the invention can be used. The CAS system includes a main computing system 322 with a display device 324, a tracking subsystem 326 in communication with the computing system and a storage device 328 in communication with the computing system in which the SSM data 318 is stored. Figure 6 is merely schematic and illustrates the major functional parts of the CAS system separately merely to facilitate explanation. In practice some or all of the parts may be provided as a single integrated system.

Computing system 322 includes various software applications which can be used by a surgeon to carry out a computer aided or assisted surgical procedure, such as an image guided surgical procedure, and can display various images of the patient and the various surgical implants, tools and instruments used by the surgeon together with visual indications of the current positions of those items and their planned positions. The surgeon can interact with the system using various input/output devices as are generally known in the art. Various of the items used by the surgeon can include markers which allow the position of those items to be tracked by the tracking subsystem which supplies tracking data to the computing system to allow images of the items and representations of their current positions to be displayed. Various types of tracking technology can be used, such as wire based and wireless technologies, such as ultrasound, infrared, electromagnetic and magnetic field based tracking technologies.

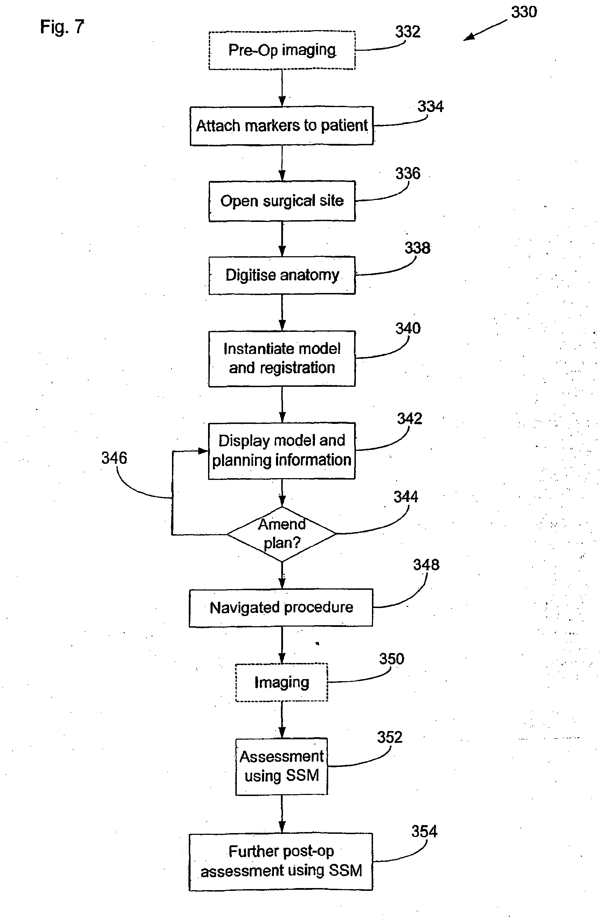

With reference to Figure 7 there is shown a flow chart illustrating a method 330 of the invention in which the SSM of the invention can be used. Figure 7 is schematic and various other steps will be carried out in practice as are known in the art, but have not been described in detail so as not to obscure the present invention.

An optional pre-operative image or images may be taken of the patient's anatomy at step 332 using any suitable imaging modality, such as X-ray, ultrasound, CT or MR scan. Then 334 markers trackable by the tracking system are attached to the patient's body parts in order to allow the location of the body parts to be tracked. In the described example, the procedure is a hip replacement procedure and so markers are attached to the femur and to the pelvis so that their position and orientation can be tracked. Then the surgical site is opened at 336 and at step 338 the patient's anatomy is digitised to provide patient specific information about the patient's anatomy. This can be done by tracking a pointer bearing a trackable marker as the tip of the pointer is run over the surface of the patient's bone, eg around the superior portion of the femur adjacent the femoral neck, and collecting the positions of a cloud of points on the surface of the femur. Then an instance of the SSM is created by fitting the captured points reflecting the patient's actual anatomy to the SSM data. This is generally carried out by minimizing a cost function between the captured points and the shape model data. An image of the instantiated model is then created and displayed at step 342. Instantiation of the SSM also registers the instantiation of the SSM in the reference frame of the tracking system as the positions of the cloud of points in the reference frame of the tracking system is known to the computer system.

Instantiation of the model as well as generating an image approximating the patient's body part also generates surgical planning information which is customised to reflect the patient's actual anatomy. The computer system takes the instantiated surgical planning point data and carries out various geometric calculations to determine the instantiated planned position and/or orientation information and then generates and displays a graphical indication of that planning information on the display screen at step 342. Position and orientation data are continuous and so can easily be handled. More care is required for the size data in order to automatically select a planned implant size. Implant sizes are generally discrete as manufacturers generally only provide implants with a fixed range of sizes, *e.g.*, small, medium or large. The instantiated size planning data may indicate, *e.g.*, an acetabular cup diameter of 37.4mm. The computer system then uses a mechanism to map the planned size to a most closely matching available implant size. For example, diameters in the range 30.0 to 34.0mm may be mapped to small, diameters in the range 34.1mm to 36.0 mm may be mapped to medium and diameters in the range 36.1 to 38.0mm may be mapped to large. Hence, it is possible to convert the continuous size values output by instantiated planning data to the discrete implant sizes available in practice.

The planning information derived from the SSM displayed to the surgeon is by way of guidance and does not have to be used. At step 344, the surgeon can enter commands to vary the planning information for example, by manually changing the position and/or orientation of the planned implant positions and/or changing the planned size of the implants. Process flow returns to step 346 at which the amended planing information is displayed relative to the model and also the deviations of the amended planning information relative to the instantiated planning information. Steps 342, 344 and 346 can be repeated until the surgeon is happy with the surgical plan and the final plan is then stored by the CAS system.

In other embodiments, amending the plan can also include selecting to use a different type of implant. In that case, SSM data for the different type of implant is retrieved by the CAS system from storage and is used to instantiate new planning data at step 340. Additionally or alternatively, in other embodiments, amending the plan can also include selecting to use a different type of surgical approach or procedure. For example, the surgeon may decide that the proposed plan is not suitable for the patient's particular anatomy, *e.g.* the acetabulum of the patient may be greatly diseased, and so a different type of surgical procedure may be more likely to result in a successful operation. In that case, SSM data for the different type of surgical procedure is retrieved by the CAS system from storage and is used to instantiate new planning data at step 340.

Additionally or alternatively, in other embodiments, amending the plan can also include selecting to change the plan based on the patient type, for example, the patient's size, weight, age, sex or race, or based on the patient's occupation or activities. For example, the surgeon may decide that the proposed plan is not suitable for the patient as they are a professional athlete and so a different type of surgical plan may be required in order to meet the patient's post operative performance requirements. In that case, SSM data for the surgical procedure appropriate for the athlete is retrieved by the CAS system from storage and is used to instantiate new planning data at step 340.

After the plan has been finalised at step 348 the surgical procedure is carried out by the surgeon using trackable implants, tools and instruments which can be navigated using the CAS system. When the surgical procedure has been completed, immediately post operative imaging can optionally be carried out at step 350, for example by capturing an X-ray image of the patient's hip joint for use in post operative audit. The instantiated SSM can be used at step 352 to assess the surgery. For example, a 2D view of the instantiated SSM image and instantiated plan can be created for the same view as that of the X-ray. Then the 2D X-ray image showing the actual positions of the implants relative to the anatomy can be compared with the 2D image generated from the instantiated SSM to compare the actual prosthetic joint with the instantiated plan. Alternatively the 2D X-ray can be registered to the instantiated 3-D shape to verify the position of the implant in 3-D.

Some further post operative assessment can be carried out later on, *e.g.* 6 months later, as indicated by step 354. A further X-ray, or other image, of the patient's hip joint can be captured and again compared with a 2D image and the plan derived from the instantiated SSM. Hence, any changes in the joint, for example movement of the implants, can be identified and monitored, for example to determine if revision surgery may be required. Step 354 can be repeated multiple times and after different periods of time, for example, annually.

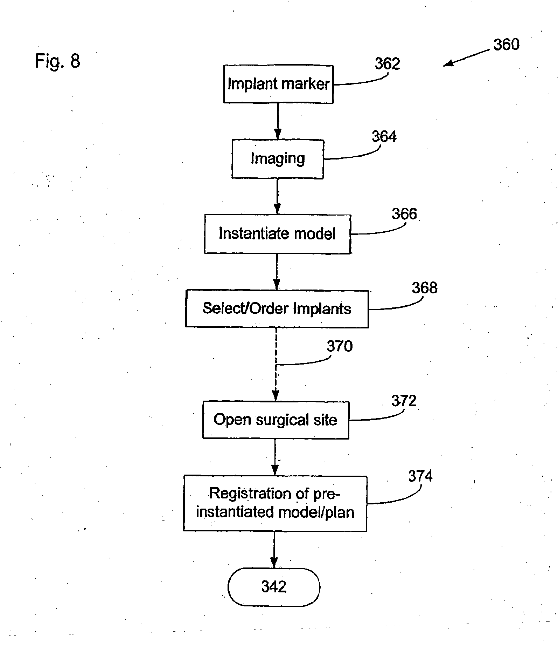

Figure 8 shows a flow chart illustrating a further method 360 of the invention in which the SSM of the invention can be used. Figure 8 is schematic and various other steps will be carried out in practice as are known in the art, but have not been described in detail so as not to obscure the present invention. Method 360 is similar to method 330 and steps 342 to 354 of method 330 can be carried out after step 374 of method 360. Method 360 differs substantially from method 330 in that it uses a non-invasive instantiation of the SSM which is carried out some time before the surgical procedure, *e.g.* as an out patient procedure. Method 360 also differs in the way registration of the SSM and plan can be achieved automatically.

At step 362 markers trackable by the CAS system are implanted in the patient's bones. Suitable markers and instruments for implanting the markers are described in International patent publication WO 2005/084572 . Then at step 364 the patient's anatomy is imaged using an imaging modality that also captures the image of the markers implanted in the bones. For example, X-ray, X-ray fluoroscopy of CT scan imaging can be used. Then at step 366, the SSM is instantiated using the image of the patients anatomy to provide the anatomy specific data to which the SSM is fitted, in a manner similar to that described above for method 330. Hence, a patient specific model of the patient's bones and a patient specific surgical plan can be generated without having to undergo any invasive surgical steps. The instantiated surgical planning information can then be used to determine both the best type of implant or implants to be used and also the correct size of the implant or implants at step 368.

Hence, prior to surgery, the implants to be used can have been pre-selected and the implants can be ordered to ensure that the correct type and size of implant is available for when the surgery is carried out. This has implications for inventory management as the hospital does not need to keep an extensive stock and can order implants as and when they are needed.

In another embodiment, the instantiated plan may show that there is no suitable standard implant available for the patient. Hence, the implant size information and information about the geometry and shape of the implant, *e.g.* a femoral stem component, can be used to create a bespoke or custom implant tailored to the patient. And this can be achieved without having to carry out any invasive surgical steps.

As indicated by dashed line 370, the implant selection and/or ordering step 368 can be carried out some time before the actual surgical procedure is begun, *e.g.* several months. The surgical procedure begins and the surgical site is opened at step 372. The already instantiated SSM and planning data are made available to the CAS system and need to be registered in the reference frame of the tracking system so that items can be navigated relative to the model and plan. If the optional marker implantation step has been done, then the instantiated model and plan can be automatically registered using a procedure similar to that described in International patent publication no WO 2005/086062.

In brief, the tracking system can determine the position of the pre-implanted markers and determine the position of the marker in the operating theatre. There is a fixed relationship between the marker and the pre-operative images and the position of the images in the operating theatre is obtained by mapping the part of the pre-operative image corresponding to the marker onto the actual position of the marker in the operating theatre. There is a fixed relationship between the pre-operative images and the instantiated SSM and so the instantiated SSM can be registered in the operating theatre by fitting the instantiated SSM to at least one of the pre-operative images used to instantiate the model.

In an alternate embodiment in which there was no pre-operative implantation of the marker, then the pre-instantiated SSM model and plan can be registered to the patient's anatomy by using a marked pointer to indicate the position of a number of anatomical features and then fitting the corresponding features in the instantiated model to the actual patient features. In a further alternate embodiment, registration can also be achieved by fitting the pre-instantiated model and plan to images captured in the operating theatre. The imaging system is calibrated so that the images can be related back to physical space and the imaging system itself is tracked so that the imaging system's coordinate system can be registered to the patient's anatomy.

The remainder of the method can be carried out similarly to steps 342 to 354 of Figure 7.

It will be appreciated that the SSM incorporating surgical planning data can be used in a number of applications which may or may not actually involve surgery being carried out. For example, as described above, instantiating the SSM can provide implant information which can be used in the customised design and manufacturing of implants. The SSM can be used in training applications, in which a new surgeon plans a surgical procedure using software on a virtual model of patient anatomy and then the planned procedure is compared to an instantiated plan which was created by using the anatomy of the virtual model. Other training applications can include a surgeon learning a new technique by using the instantiated planning data to assist in carrying out a procedure with which they are less familiar.

The instantiated planning information can also be used as part of quality control or quality assurance. For example, the plan created by a surgeon can be compared with an instantiated plan prior to any cuts being made in order to ensure that the surgeon's own plan is within certain acceptable tolerances. Alternatively, or additionally, the actual plan used during surgery, or the resulting implant sizes and placements, can be compared with an instantiated plan to assess how closely the surgeons technique matches the best practice or technique embodied in the instantiated plan. The instantiated plans can also be saved and archived, so that a surgeon can monitor and assess their own performance and to provide an historical record, *e.g.* for audit purposes.

Generally, embodiments of the present invention employ various processes involving data stored in or transferred through one or more computer systems. Embodiments of the present invention also relate to an apparatus for performing these operations. This apparatus may be specially constructed for the required purposes, or it may be a general-purpose computer selectively activated or reconfigured by a computer program and/or data structure stored in the computer. The processes presented herein are not inherently related to any particular computer or other apparatus. In particular, various general-purpose machines may be used with programs written in accordance with the teachings herein, or it may be more convenient to construct a more specialized apparatus to perform the required method steps. A particular structure for a variety of these machines will appear from the description given below.

In addition, embodiments of the present invention relate to computer readable media or computer program products that include program instructions and/or data (including data structures) for performing various computer-implemented operations. Examples of computer-readable media include, but are not limited to, magnetic media such as hard disks, floppy disks, and magnetic tape; optical media such as CD-ROM disks; magnetooptical media; semiconductor memory devices, and hardware devices that are specially configured to store and perform program instructions, such as read-only memory devices (ROM) and random access memory (RAM). The data and program instructions of this invention may also be embodied on a carrier wave or other transport medium. Examples of program instructions include both machine code, such as produced by a compiler, and files containing higher level code that may be executed by the computer using an interpreter.

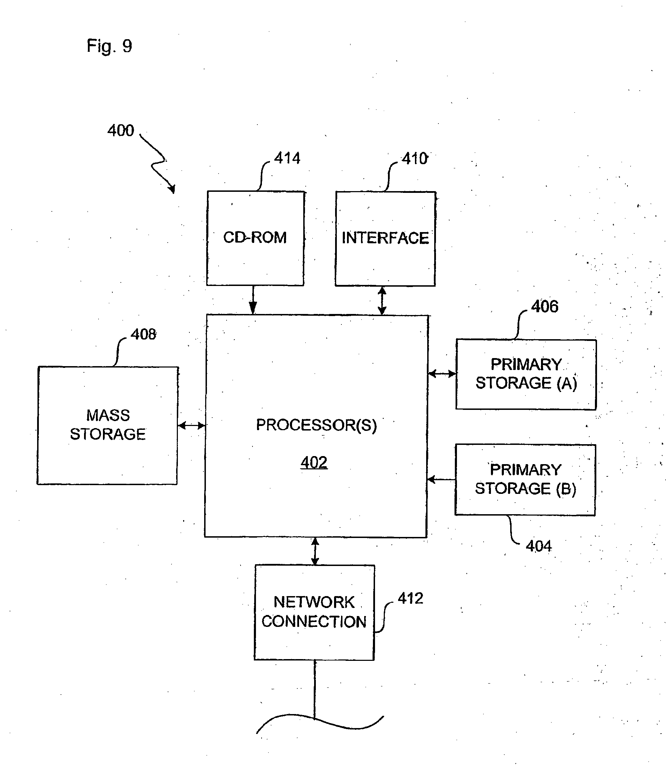

Figure 9 illustrates a typical computer system that, when appropriately configured or designed, can serve as the data processing apparatus or computer of the CAS system according to the invention. The data processing apparatus or computer 400 includes any number of processors 402 (also referred to as central processing units, or CPUs) that are coupled to storage devices including primary storage 406 (typically a random access memory, or RAM), primary storage 404 (typically a read only memory, or ROM). CPU 402 may be of various types including microcontrollers and microprocessors such as programmable devices (e.g., CPLDs and FPGAs) and unprogrammable devices such as gate array ASICs or general purpose microprocessors. As is well known in the art, primary storage 404 acts to transfer data and instructions uni-directionally to the CPU and primary storage 406 is used typically to transfer data and instructions in a bidirectional manner. Both of these primary storage devices may include any suitable computer-readable media such as those described above. A mass storage device 408 is also coupled bi-directionally to CPU 402 and provides additional data storage capacity and may include any of the computer-readable media described above. Mass storage device 408 may be used to store programs, data and the like and is typically a secondary storage medium such as a hard disk. It will be appreciated that the information retained within the mass storage device 408, may, in appropriate cases, be incorporated in standard fashion as part of primary storage 406 as virtual memory. A specific mass storage device such as a CD-ROM 414 may also pass data uni-directionally to the CPU.

CPU 402 is also coupled to an interface 410 that connects to one or more input/output devices such as such as video monitors, track balls, mice, keyboards, microphones, touch-sensitive displays, transducer card readers, magnetic or paper tape readers, tablets, styluses, voice or handwriting recognizers, or other well-known input devices such as, of course, other computers. Finally, CPU 402 optionally may be coupled to an external device such as a database or a computer or telecommunications network using an external connection as shown generally at 412. With such a connection, it is contemplated that the CPU might receive information from the network, or might output information to the network in the course of performing the method steps described herein.

Although the above has generally described the present invention according to specific processes and apparatus, the present invention has a much broader range of applicability. In particular, aspects of the present invention is not limited to any particular kind of orthopaedic procedure and can be applied to virtually any method in which information about the position of a component relative to a patient's anatomy can be of use. Thus, in some embodiments, the techniques of the present invention could be used to plan the positions and/or sizes and/or types of components to be used relative to bony and/or soft structures, such as tissues, ligaments, organs, etc., either pre-, intra- or post operatively. One of ordinary skill in the art would recognize other variants, modifications and alternatives in light of the foregoing discussion.

**Claims**

1. A computer implemented method (300) for creating a statistical shape model incorporating surgical planning information for a body part, comprising:

   generating anatomical data (306) representing the anatomical shape of the body part from images (302) of a plurality of training subjects;
   generating planning data (306) which can be used to represent at least one planning property for a surgical procedure from the images of the plurality of training subjects, wherein the planning property includes the position or orientation of a surgical instrument or a prosthetic component to be implanted using the surgical procedure; and
   creating (316) a statistical shape model (318) from the anatomical data and the planning data and which incorporates the planning data.

2. The method of claim 1, wherein the statistical shape model is a point distribution model and wherein the planning data is defined in terms of a number of points.

3. The method of claim 1, wherein the at least one planning property also includes the size of the prosthetic component.

4. The method of claim 1, wherein the at least one planning property also includes the size or type of the surgical instrument to be used in the surgical procedure.

5. The method of claim 1, wherein the anatomical data and the planning data are represented as a $3(n + m)$ element vector in which $n$ is the number of points defining the surface of the body part and $m$ is the number of points representing the planning property.

6. The method of claim 1, wherein principal component analysis of training data sets comprising the anatomical data and the planning data for plurality of training subjects is used to create the statistical shape model.

7. The method of claim 1, wherein the surgical procedure is a shoulder replacement, a spinal procedure, a trauma procedure or a fracture procedure.

8. The method of claim 1, wherein the surgical procedure comprises a hip replacement operation and wherein the planning property includes the position and/or orientation of an acetabular cup relative to the pelvis and/or the position of a femoral head component.

9. The method of claim 1, wherein the surgical procedure comprises a knee replacement operation and wherein the planning property includes the position and/or size and/or orientation of a tibial component and/or the position and/or size and/or orientation of a femoral component.

10. The method of claim 1, wherein the images of a plurality of training subjects are images including an implanted prosthesis.

11. The method of claim 1, wherein the plurality of training subjects are selected by age, gender, ethnicity, race or any combination thereof.

**12.** The method of claim 1, wherein the plurality of training subjects are selected by their having a particular condition, disease or other property affecting their anatomy.

**13.** The method of claim 1, wherein the plurality of training subjects are grouped by the type of surgical procedure, the type of implant used in a surgical procedure, the type of component used in a surgical procedure, the surgeon or group of surgeons that carried out the surgical procedure.

**14.** A computer implemented method (104) of instantiating a statistical shape model and automatically generating surgical planning information, comprising:

creating a statistical shape model according to the method of any of claims 1 to 13; and
instantiating (116) a model of an actual patient using information derived (114) from the actual anatomy of the patient, whereby instantiating the model automatically generates surgical planning data (118) which directly or indirectly provides surgical planning information adapted for the patients actual anatomy.

**15.** An apparatus for creating a statistical shape model incorporating surgical planning information for a body part, comprising a data processing device and a memory storing computer program instructions which can configure the data processing device to carry out the method of any preceding claim.

**16.** A computer program product comprising a computer readable medium bearing computer program code executable by a data processing device to provide the method of any of claims 1 to 14 or the apparatus of claim 15.


**Patentansprüche**

**1.** Computerimplementiertes Verfahren (300) zum Erzeugen eines statistischen Formmodells, das eine chirurgische Planungsinformation für einen Körperteil einbezieht, umfassend:

Erzeugen von anatomischen Daten (306), welche die anatomische Form des Körperteils darstellen, aus Bildern (302) einer Mehrzahl von Trainingssubjekten,
Erzeugen von Planungsdaten (306), welche dazu verwendet werden können,
zumindest eine Planungseigenschaft für einen chirurgischen Eingriff darzustellen, aus den Bildern der Mehrzahl von Trainingssubjekten, wobei die Planungseigenschaft die Position oder Orientierung eines chirurgischen Instruments oder einer unter Verwendung des chirurgischen Eingriffs zu implantierenden Prothesenkomponente enthält, und
Erzeugen (316) eines statistischen Formmodells (318) aus den anatomischen Daten und den Planungsdaten und welches die Planungsdaten einbezieht.

**2.** Verfahren nach Anspruch 1, wobei das statistische Formmodell ein Punktverteilungsmodell ist, und wobei die Planungsdaten in der Form einer Anzahl von Punkten definiert sind.

**3.** Verfahren nach Anspruch 1, wobei die zumindest eine Planungseigenschaft auch die Größe der Prothesenkomponente enthält.

**4.** Verfahren nach Anspruch 1, wobei die zumindest eine Planungseigenschaft auch die Größe oder den Typ des bei dem chirurgischen Eingriff zu verwendenden chirurgischen Instruments enthält.

**5.** Verfahren nach Anspruch 1, wobei die anatomischen Daten und die Planungsdaten als ein $3(n+m)$-Elementenvektor dargestellt sind, wobei $n$ die Anzahl von Punkten, welche die Oberfläche des Körperteils definieren, ist, und wobei $m$ die Anzahl von Punkten, welche die Planungseigenschaft darstellen, ist.

**6.** Verfahren nach Anspruch 1, wobei eine Hauptkomponentenanalyse von Trainingsdatenmengen, welche die anatomischen Daten und die Planungsdaten für die Mehrzahl von Trainingssubjekten umfassen, dazu verwendet wird, das statistische Formmodell zu erzeugen.

**7.** Verfahren nach Anspruch 1, wobei der chirurgische Eingriff ein Schulterersatz, ein Wirbelsäuleneingriff, ein Traumabehandlungsverfahren oder ein Bruchbehandlungsverfahren ist.

**8.** Verfahren nach Anspruch 1, wobei der chirurgische Eingriff eine Hüftersatzoperation umfasst, und wobei die Planungseigenschaft die Position und/oder Orientierung einer Hüftgelenkspfanne relativ zu dem Becken und/oder die Position einer Femurkopfkomponente enthält.

**9.** Verfahren nach Anspruch 1, wobei der chirurgische Eingriff eine Knieersatzoperation umfasst, und wobei die Planungseigenschaft die Position und/oder Größe und/oder Orientierung einer Tibiakomponente und/oder die Position und/oder Größe und/oder Orientierung einer Femurkomponente enthält.

**10.** Verfahren nach Anspruch 1, wobei die Bilder einer Mehrzahl von Trainingssubjekten Bilder sind, die eine implantierte Prothese enthalten.

**11.** Verfahren nach Anspruch 1, wobei die Mehrzahl von Trainingssubjekten anhand von Alter, Geschlecht, Ethnizität, Rasse oder einer beliebigen Kombination davon ausgewählt ist.

**12.** Verfahren nach Anspruch 1, wobei die Mehrzahl von Trainingssubjekten anhand dessen ausgewählt ist, dass sie einen bestimmten Zustand, eine bestimmte Krankheit oder eine andere Eigenschaft, welche ihre Anatomie beeinflusst, aufweisen.

**13.** Verfahren nach Anspruch 1, wobei die Mehrzahl von Trainingssubjekten nach dem Typ eines chirurgischen Eingriffs, dem Typ des bei einem chirurgischen Eingriff verwendeten Implantats, dem Typ der bei einem chirurgischen Eingriff verwendeten Komponente, dem Chirurgen oder der Gruppe von Chirurgen, welche den chirurgischen Eingriff durchgeführt haben, gruppiert sind.

**14.** Computerimplementiertes Verfahren (104) zum Instanziieren eines statistischen Formmodells und automatischen Erzeugen einer chirurgischen Planungsinformation, umfassend:

Erzeugen eines statistischen Formmodells gemäß dem Verfahren nach einem der Ansprüche 1 bis 13 und Instanziieren (116) eines Modells eines tatsächlichen Patienten, wobei eine von der tatsächlichen Anatomie des Patienten abgeleitete Information (114) verwendet wird, wodurch das Instanziieren des Modells automatisch chirurgische Planungsdaten (118) erzeugt, welche direkt oder indirekt eine für die tatsächliche Anatomie des Patienten angepasste chirurgische Planungsinformation bereitstellen.

**15.** Einrichtung zum Erzeugen eines statistischen Formmodells, das eine chirurgische Planungsinformation für einen Körperteil einbezieht, umfassend eine Datenverarbeitungsvorrichtung und einen Speicher, der Computerprogramminstruktionen, welche die Datenverarbeitungsvorrichtung zum Ausführen des Verfahrens nach einem der vorhergehenden Ansprüche konfigurieren können, speichert.

**16.** Computerprogrammprodukt, umfassend ein computerlesbares Medium, das einen Computerprogrammcode trägt, der von einer Datenverarbeitungsvorrichtung ausführbar ist, um das Verfahren nach einem der Ansprüche 1 bis 14 oder die Einrichtung nach Anspruch 15 bereitzustellen.

**Revendications**

**1.** Procédé mis en oeuvre par ordinateur (300) pour créer un modèle de forme statistique incorporant des informations de programmation chirurgicale pour une partie corporelle, comprenant :

produire des données anatomiques (306) représentant la forme anatomique de la partie corporelle à partir d'images (302) d'une pluralité de sujets d'expérience ;
produire des données de planification (306) qui peuvent être utilisées pour représenter au moins une propriété de planification pour une procédure chirurgicale à partir des images de la pluralité de sujets d'expérience, où la propriété de planification inclut la position ou orientation d'un instrument chirurgical ou d'un composant de prothèse à implanter en utilisant la procédure chirurgicale ; et
créer (316) un modèle de forme statistique (318) à partir des données anatomiques et des données de planification et qui incorpore les données de planification.

**2.** Procédé selon la revendication 1, dans lequel le modèle de forme statistique est un modèle de distribution de points, et où les données de planification sont définies en termes d'un nombre de points.

3. Procédé selon la revendication 1, dans lequel la au moins une propriété de planification inclut également la taille du composant de prothèse.

4. Procédé selon la revendication 1, dans lequel la au moins une propriété de planification inclut également la taille ou le type de l'instrument chirurgical à utiliser dans la procédure chirurgicale.

5. Procédé selon la revendication 1, dans lequel les données anatomiques et les données de planification sont représentées comme un vecteur d'élément $3(n + m)$ dans lequel $n$ est le nombre de points définissant la surface de la partie corporelle et m est le nombre de points représentant la propriété de planification.

6. Procédé selon la revendication 1, dans lequel l'analyse de composant principal d'ensembles de données d'entraînement comprenant les données anatomiques et les données de planification pour une pluralité de sujets d'expérience est utilisée pour créer le modèle de forme statistique.

7. Procédé selon la revendication 1, dans lequel la procédure chirurgicale est un remplacement d'épaule, une procédure spinale, une procédure de trauma ou une procédure de fracture.

8. Procédé selon la revendication 1, dans lequel la procédure chirurgicale comprend une opération de remplacement de la hanche, et où la propriété de planification comprend la position et/ou l'orientation d'une cotyle relativement au bassin et/ou la position d'un composant de tête fémorale.

9. Procédé selon la revendication 1, dans lequel la procédure chirurgicale comprend une opération de remplacement du genou, et où la propriété de planification inclut la position et/ou la taille et/ou l'orientation d'un composant tibial et/ou la position et/ou la taille et/ou l'orientation d'un composant fémoral.

10. Procédé selon la revendication 1, dans lequel les images d'une pluralité de sujets d'expérience sont des images incluant une prothèse implantée.

11. Procédé selon la revendication 1, dans lequel la pluralité de sujets d'expérience sont sélectionnés d'après l'âge, le sexe, l'ethnicité, la race ou leurs combinaisons.

12. Procédé selon la revendication 1, dans lequel la pluralité de sujets d'expérience sont sélectionnés selon leur condition, maladie ou autre propriété particulière affectant leur anatomie.

13. Procédé selon la revendication 1, dans lequel la pluralité de sujets d'expérience sont groupés selon le type de procédure chirurgicale, le type d'implant utilisé dans une procédure chirurgicale, le type de composant utilisé dans une procédure chirurgicale, le chirurgien ou le groupe de chirurgiens qui ont exécuté la procédure chirurgicale.

14. Procédé mis en oeuvre par ordinateur (104) pour instancier un modèle de forme statistique et pour produire automatiquement des informations de planification chirurgicale, comprenant :

   créer un modèle de forme statistique selon le procédé selon l'une quelconque des revendications 1 à 13 ; et instancier (116) un modèle d'un patient réel utilisant des informations dérivées (114) de l'anatomie réelle du patient, moyennant quoi l'instanciation du modèle produit automatiquement des données de planification chirurgicale (118) qui fournissent directement ou indirectement des informations de planification chirurgicale conçues pour l'anatomie réelle du patient.

15. Appareil pour créer un modèle de forme statistique incorporant des informations de planification chirurgicales pour une partie corporelle, comprenant un dispositif de traitement de données et une mémoire stockant des instructions de programme d'ordinateur qui peuvent configurer le dispositif de traitement des données afin d'exécuter le procédé selon l'une quelconque des revendications précédentes.

16. Produit programme d'ordinateur comprenant un support lisible par ordinateur supportant un code programme d'ordinateur apte à être exécuté par un dispositif de traitement de données pour la mise en oeuvre du procédé selon l'une quelconque des revendications 1 à 14 ou de l'appareil de la revendication 15.

Fig. 1

100

Begin

Create SSM
incorporating surgical
planning information — 102

Instantiate and use SSM
incorporating surgical
planning information — 104

Done

Fig. 2

110

Incorporate surgical
plan info into
statistical shape
model — 112

Digitise body
part points — 114

Fit shape model to
body part — 116

Surgical Plan, e.g.
cup position - point + vector
femoral head position - 6 D of F — 118

FIG. 3

FIG. 4

Fig. 5

Fig. 6

Fig. 7

Fig. 8

360

| Implant marker | 362

↓

| Imaging | 364

↓

| Instantiate model | 366

↓

| Select/Order Implants | 368

370

↓

| Open surgical site | 372

↓

| Registration of pre-instantiated model/plan | 374

↓

( 342 )

Fig. 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0203304 A
- WO 2004008943 A
- WO 2005084572 A
- WO 2005086062 A

**Non-patent literature cited in the description**

- Computer assisted spinal surgery using anatomy based registration. **LAVALLEE S et al.** Registration for Computer Integrated Surgery: Methodology. State of the Art, 1995, 425-449
- **CHAN C S K et al.** Cadaver validation of the use of ultrasound for 3D model instantiation of bony anatomy in image guided orthopaedic surgery. *Proceedings of MICCAI,* 2004
- **P BESL ; N MCKAY.** A method for registration of 3D shapes. *IEEE Trans Pattern Anal Machine Intell,* 1992, vol. 14, 239-256
- **EDWARDS et al.** *Proc CAOS,* 2002
- **COOTES, T. F. ; TAYLOR, C. J. ; COOPER, D. H. ; GRAHAM, J.** Training Models of Shape from Sets of Examples. *Proceedings of British Machine Vision Conference,* 1992, 9-18
- **MORRISON, D.F.** ultivariate Statistical Methods. McGraw-Hill, 1990, 313-322
- **RUECKERT, D. ; FRANGI, A. F. ; SCHNABEL, J. A.** *Automatic Construction of 3-D Statistical Deformation Models of the Brain Using Nonrigid Registration,* 2003, 1014-1025